# EUROPÄISCHE PATENTSCHRIFT

(11) **EP 0 783 314 B1**
(45) Veröffentlichungstag und Bekanntmachung des Hinweises auf die Patenterteilung: **28.05.2003**
(21) Anmeldenummer: 95935387.1
(22) Anmeldetag: 27.09.1995
(51) Int. Cl.: A61K 35/42, A61K 31/57, A61K 31/685, A61K 38/17, A61K 31/58, A61P 11/00

(54) **ZUSAMMENSETZUNGEN ZUR BEHANDLUNG VON IRDS UND ARDS, DIE MINDESTENS EIN GLUCOCORTICOSTEROID IN KOMBINATION MIT EINEM LUNGEN SURFACTANT ENTHALTEN**
COMPOSITIONS CONTAINING AT LEAST ONE GLUCOCORTICOID IN COMBINATION WITH A PULMONARY SURFACTANT FOR THE TREATMENT OF IRDS AND ARDS
COMPOSITIONS CONTENANT AU MOINS UN GLUCOCORTICOIDE EN ASSOCIATION AVEC UN SURFACTANT PULMONAIRE POUR LE TRAITEMENT DU SYNDROME DE DETRESSE RESPIRATOIRE DU NOURRISSON ET DES INSUFFISANCES RESPIRATOIRES AIGUES

(30) Priorität: 28.09.1994 DE 4434629
(43) Veröffentlichungstag der Anmeldung: 16.07.1997
(73) Patentinhaber: ALTANA Pharma AG, 78467 Konstanz (DE)
(72) Erfinder: GERMANN, Paul-Georg, 21255 Tostedt (DE); EISTETTER, Klaus, 78465 Konstanz (DE); KILIAN, Ulrich, 78479 Reichenau (DE); HÄFNER, Dietrich, 78464 Konstanz (DE)
(86) Internationale Anmeldenummer: EP9503816
(87) Internationale Veröffentlichungsnummer: WO96009831

(56) Entgegenhaltungen:
- WO-A-90/07469
- EXP. LUNG RES., 1990, 311-21, FIASCONE, JOHN M. ET AL 'Corticosteroids and intratracheal surfactant both alter the distribution between the airways and lung tissue of intratracheally administered radiolabeled phosphatidylcholine in the preterm rabbit'
- J APPL PHYSIOL, 67 (4). 1989. 1377-1382., GLADSTONE I M ET AL 'ANTENATAL STEROIDS POSTNATAL SURFACTANT AND PULMONARY FUNCTION IN PREMATURE RABBITS'
- JOINT MEETING OF THE AMERICAN PEDIATRIC SOCIETY AND THE SOCIETY FOR PEDIATRIC RESEARCH, ANAHEIM, CALIFORNIA, USA, APRIL 27-30, 1987. PEDIATR RES, 21 (4 PART 2). 1987. 458A., KWONG M ET AL 'SYNERGISTIC RESPONSE OF ANTENATAL BETAMETHASONE AND TRACHEAL INSTILLATION OF CALF LUNG SURFACTANT EXTRACT CLSE AT BIRTH'
- PEDIATRICS, 1994, 93/5 (730-736), USA, KARI M.A. ET AL 'Prenatal dexamethasone treatment in conjunction with rescue therapy of human surfactant: A randomized placebo-controlled multicenter study'
- ANN. MED., 1993, 25/3 (285-288), UNITED KINGDOM, ROBERTSON B. 'Corticosteroids and surfactant for prevention of neonatal RDS'

## Beschreibung

### Technisches Gebiet

Die Erfindung betrifft eine neue Zusammensetzung zur Behandlung von IRDS und ARDS.

### Stand der Technik

Es ist bekannt, daß eine Behandlung mit Glucocorticosteroiden (GCS) bei Muttern, die zu Frühgeburten neigen, die Folgen des Infant Respiratory Distress Syndromes (=IRDS) bei deren Babys mildern können (z. B. H. R. Gamsu, B. M. Mullinger, P. Donai and C. H. Dash: Antenatal administration of Betamethasone to prevent respiratory distress syndrome in preterm infants: report of a UK multicentre trial, Brit. J. Obst. Gyn. 1989, 96:410-10; Review: A. N. Papageorgiou and L. Stern: J. Perinat. Med. 1986, 14:75-86). Hierzu erhalten die Mütter GCS. Sodann wird versucht, die Geburt um mindestens 24 Stunden zu verzögern, um die durch GCS bedingte Lungenreifung sich ausprägen zu lassen. Ebenfalls seit Jahren erhalten frühgeborene Kinder Lungensurfactants (LSF) zur Vorbeugung und/oder Behandlung des IRDS durch intratracheale oder intrabronchiale Instillation verabreicht (A. Jobe and M. Ikegami: Surfactant for the treatment of respiratory distress syndrome. Am. Rev. Respir. Dis. 1987, 136:1256-75; M. S. Reynolds and K. A. Wallander: Use of surfactant in the prevention and treatment of neonatal respiratory distress syndrome, Clin. Pharm. 1989, 8:559-76). In letzter Zeit mehren sich die Pivotal-Studien in denen LSF zur Therapie des Acute Respiratory Distress Syndrome (ARDS) anderer Genese mit Erfolg eingesetzt wird (Übersicht z. B. B. Lachmann, D. Gommers and E. P. Eijking: Exogenous surfactant therapy in adults, Atemw.-Lungenkrkh. 1993, 19:581-91; T. J. Gregory et al.: Survanta supplementation in patients with acute respiratory distress syndrome (ARDS), Am. J. Respir. Crit. Care Med. 1994, 149:A567). Corticosteroide werden beim ARDS mit wenig Erfolg eingesetzt (G. R. Bernard et al.: High-dose corticosteroids in patients with the adult respiratory distress syndrome, N. Engl. J. Med. 1987, 317:1565-70).

### Beschreibung der Erfindung

Es wurde nun überraschend festgestellt, daß durch die Verabreichung einer Kombination von Glucocorticosteroiden und Lungensurfactants ein synergistischer Effekt bei der Behandlung von IRDS und ARDS erreicht werden kann.

Gegenstand der Erfindung ist deshalb eine Zusammensetzung zur Behandlung von IRDS und ARDS enthaltend mindestens ein Glucocorticosteroid und einen Lungensurfactant unter Ausschluss von Kombinationen, die wirksame Mengen an γ-Interferon oder TNF enthalten.

Weitere Ausgestaltungen der Erfindung ergeben sich aus den Patentansprüchen.

Als Glucocorticosteroide kommen solche in Frage, die sich zur Applikation in der Lunge eignen. Beispielsweise seien Betamethason, Budesonide, Methylprednisolon, Dexamethason und Ciclesonide genannt.

Unter Lungensurfactants werden erfindungsgemäß die zahlreichen bekannten Zusammensetzungen verstanden, die die Funktion des natürlichen Lungensurfactants besitzen. Es handelt sich hierbei um Zusammensetzungen von insbesondere Phospholipiden, die unter anderem noch Lungensurfactant-Proteine enthalten können. An Handelsprodukten sind zu nennen Curosurfoe (Serono, Pharma GmbH, 85716 Unterschleißheim), ein hochgereinigtes natürliches Surfactant aus homogenisierten Schweinelungen, Survantaoe (Abbott GmbH, Wiesbaden) und Alveofactoe (Dr. Karl Thomae GmbH Biberach), beides Extrakte aus Rinderlungen, sowie Exosurfoe (Deutsche Wellcome GmbH, Burgwedel), ein synthetisches Phospholipid mit Hilfsstoffen. Als Lungensurfactant-Proteine kommen sowohl die aus natürlichen Quellen, wie beispielsweise Lungen-Lavage oder Extraktion aus Fruchtwasser, gewonnenen als auch die gentechnisch hergestellten Proteine in Frage. Erfindungsgemäß sind insbesondere die mit SP-B und SP-C bezeichneten Lungensurfactant-Proteine und deren modifizierten Derivate von Interesse. Die Aminosäuresequenzen dieser Lungensurfactant-Proteine, ihre Isolierung bzw. gentechnologische Herstellung sind bekannt (WO-86/03408, EP-A-0251449, WO-89/04326, WO-87/06943, WO-88/03170, EP-A-0368823 und EP-A-0348967). In EP-B-0100910, EP-A-0110498, EP-B-0119056, EP-B-0145005 und EP-B-0286011 sind Phospholipid-Zusammensetzungen mit und ohne Lungensurfactant-Proteine beschrieben, die beispielsweise als Komponenten der erfindungsgemäßen Zubereitungen in Frage kommen.

Das Dokument US 4 944 941 beschreibt Kombinationen, die γ-Interferon und/oder TNF enthalten, zur Behandlung von IRDS.

Die erfindungsgemäßen Zusammensetzungen werden entweder in Pulverform zur inhalativen Verabreichung oder in flüssiger Form zur intratrachealen oder intrabronchialen Verabreichung zur Verfügung gestellt. Eine Pulverform erhält man, indem man flüssige Lungensurfactant-Zubereitungen beispielsweise vor oder nach Zugabe des Glucocorticosteroids lyophilisiert und anschließend mikronisiert. Erfindungsgemäße Zusammensetzungen enthalten 1 bis 30 Gewichtsprozent Glucocorticosteroid (je nach Wirkstärke des GCS; eine Tabelle mit relativen Wirkstärken von Glucocorticosteroiden findet sich bei Goodman/Gillman, Pharmacological Basis of Therapeutics, Pergamon Press, Seite 1447, 8th Ed.)und 15 bis 95 Gewichtsprozent Lungensurfactant bezogen auf die Trockenmasse (z. B. Betamethasone 7 % und LSF 92 % oder Methylprednisolon 37 % und LSF 63 %).

Die erfindungsgemäßen Zubereitungen werden 3 bis 4 mal täglich über 2 bis 4 Tage verabreicht. Beispielsweise werden Zubereitungen enthaltend 4 mg Betamethason und 50 mg Phospholipide 6mal im Abstand von 6 Stunden inhalativ oder intratracheal oder intrabronchial verabreicht.

### Pharmakologie

Ausgewachsene (adulte) Sprague Dawley Ratten werden künstlich mit reinem Sauerstoff und einem positiv endexspiratorischen Druck ( = PEEP; um eine Oxygenierung der Ratten zu gewährleisten) beatmet und so oft lavagiert, bis ihr eigener LSF ausgewaschen ist (B. Lachmann, B. Robertson and J. Vogel: In vivo lung-lavage as an experimental model of the respiratory distress syndrome, Acta Anesth. Scand. 1980, 24:231-6; D. Häfner, U. Kilian and R. Beume: Comparison of four lung surfactant preparations in an animal model of adult respiratory distress syndrome (ARDS). Am. Rev. Respir. Dis. 1993, 147:A719; D. Häfner, P.-G. Germann, D. Hauschke, Pulmonary Pharmacology (1994)7, 319-332.). Dies äußert sich dadurch, daß die Tiere von Vorwerten eines arteriellen Sauerstoffpartialdruckes (PaO2) von 500 - 550 mmHg (bei reiner Sauerstoffbeatmung und PEEP) auf Werte von 50 - 110 mmHg abfallen. Tiere der Kontrollgruppe, die nicht mit LSF behandelt werden, bleiben mit ihrem PaO2 über die Beobachtungszeit bei diesen niedrigen Werten. Fünf Minuten nachdem der PaO2 auf diese Werte abgefallen ist, wird LSF bzw. LSF zusammen mit einem Glucocorticosteroid intratracheal instilliert. Die Blutgase werden nach 5, 30, 60, 90 und 120 Minuten nach Instillation bestimmt. Danach wird der PEEP von 8 auf 6 cm H₂O erniedrigt (erste PEEP-Erniedrigung). Weitere 15 Minuten später wird der PEEP auf 3 cm H₂O reduziert (zweite PEEP-Erniedrigung). Die Blutgase werden jeweils 5 Minuten nach allen beiden PEEP-Erniedrigungen bestimmt.

in der nachfolgenden Tabelle 1 sind in Zeile A die Mittelwerte (± Standardabweichung) des PaO2 in mmHg über den Zeitraum von 5 bis 120 Minuten (konstanter PEEP von 8 cm H₂O) nach der intratrachealen Instillation angegeben. Zeile B zeigt die Mittelwerte (± Standardabweichung) des PaO2 nach der ersten PEEP-Erniedrigung nach der intratrachealen Instillation. Aus der Zeile C sind die Mittelwerte des PaO2 (± Standardabweichung) während der zweiten PEEP-Erniedrigung nach der Instillation zu entnehmen. Aus der Tabelle ist ersichtlich, daß die alleinige Gabe des Glucocorticosteroids (hier Budesonide) keinen Einfluß auf den PaO2 hat. Dies ergibt sich durch Vergleich mit den unbehandelten Kontrolltieren. Die Gabe von LSF (25 oder 100 mg/kg) führt zu einer Anhebung des PaO2. Der Zusatz von 600 µg Budesonid zur jeweiligen LSF-Dosierung verbessert die PaO2-Werte signifikant gegenüber den jeweiligen LSF-Dosierungen. Daraus ergibt sich, daß die gemeinsame Gabe von Glucocorticosteroiden und LSF zu einer unerwarteten überadditiven Wirkung führt. Es ist daher möglich, einen Teil des sehr teuren LSF einzusparen, oder aber eine verstärkte Wirkung jeder einzelnen Komponente zu erhalten.

**TABELLE 1:**

| | Kontrolle | Budesonide 600 µg/kg | LSF 25 mg/kg | LSF 25 mg/kg + Budesonide 600 µg/kg | LSF 100 mg/kg | LSF 100 mg/kg + Budesonide 600 µg/kg |
|---|---|---|---|---|---|---|
| A | 82 ± 30 | 61 ± 17 | 396 ± 49 | 453 ± 50 | 496 ± 35 | 525 ± 16 |
| B | 77 ± 23 | 96 | 316 ± 91 | 437 ± 71 | 461 ± 72 | 533 ± 25 |
| C | 50 ± 8 | 58 | 103 ± 63 | 251 ± 156 | 170 ± 127 | 341 ± 103 |

Die im Anschluß an den Versuch durchgeführten histologischen Aufarbeitungen der Lungen dieser Tiere zeigen eine starke Bildung von sogenannten hyalinen Membranen (HM) und einen starken Einstrom an Entzündungszellen (z. B. polymorphkernige neutrophile Leukozyten (=PMNLl) als Ausdruck der Entwicklung eines akuten Atemnot-Syndroms.

Bei der Untersuchung erfindungsgemäßer Zubereitungen enthaltend Dexamethason oder Ciclesonide und ein Phospholipid-Gemisch mit oder ohne Surfactant-Proteinen an diesem Modell wurde gefunden, daß sich die Oxygenierung und die histologischen Veränderungen (Hemmung der Bildung von HM und Hemmung des Einstromes von PMNL) im Vergleich zu der alleinigen Gabe von LSF oder GCS überadditiv verbessern. Es ergibt sich hieraus, daß durch diesen nicht zu erwartenden synergistischen Effekt die Behandlung des IRDS und ARDS verkürzt und die mit diesen Syndromen einhergehende hohe Mortalität gesenkt werden können.

## Patentansprüche

1. Verwendung einer Kombination von Lungensurfactant und mindestens einem Glucocorticosteroid, unter Ausschluss von Kombinationen, die wirksame Mengen an γ-Interferon oder TNF enthalten, zur Herstellung eines Arzneimittels zur Behandlung von IRDS und ARDS.

2. Verwendung nach Anspruch 1, **dadurch gekennzeichnet, dass** die Kombination aus Lungensurfactant und mindestens einem Glucocorticosteroid besteht.

3. Verwendung nach Anspruch 1 oder 2, **dadurch gekennzeichnet, daß** als Glucocorticosteroid Betamethason, Methylprednisolon, Dexamethason und/oder Ciclesonide enthalten sind.

4. Verwendung nach Anspruch 1 oder 2, **dadurch gekennzeichnet, daß** als Lungensurfactant Gemische von Phospholipiden enthalten sind.

5. Verwendung nach Anspruch 4, **dadurch gekennzeichnet, daß** in natürlichen Lungensurfactants vorkommende Phospholipide enthalten sind.

6. Verwendung nach Anspruch 4, **dadurch gekennzeichnet, daß** zusätzlich Lungensurfactant-Proteine enthalten sind.

7. Verwendung nach Anspruch 6, **dadurch gekennzeichnet, daß** SP-B und/oder SP-C und/oder deren modifizierte Derivate enthalten sind.

8. Verwendung nach Anspruch 5, **dadurch gekennzeichnet, daß** durch Lungen-Lavage gewonnene Lungensurfactants enthalten sind.

9. Verwendung nach Anspruch 1 oder 2, wobei es sich um ein Arzneimittel in Pulverform zur inhalativen Verabreichung handelt.

10. Verwendung nach Anspruch 1 oder 2, wobei es sich um ein Arzneimittel in flüssiger Form zur intratrachealen oder intrabronchialen Verabreichung handelt.

## Claims

1. Use of a combination of lung surfactant and at least one glucocorticosteroid, excluding combinations containing effective amounts of γ-Interferon or TNF, for the production of a medicament for the treatment of lRDS and ARDS.

2. Use according to claim 1, **characterized in that** the combination consists of lung surfactant and at least one glucocorticosteroid.

3. Use according to claim 1 or 2, **characterised in that** as glucocorticosteroid betamethasone, methylprednisolone, dexamethasone and/or ciclesonide are contained.

4. Use according to claim 1 or 2, **characterised in that** as lung surfactant mixtures of phospholipids are contained.

5. Use according to claim 4, **characterised in that** phospholipids present in natural lung surfactant are contained.

6. Use according to claim 4, **characterised in that** lung surfactant proteins are contained additionally.

7. Use according to claim 6, **characterised in that** SP-B and/or SP-C and/or their modified derivatives are contained.

8. Use according to claim 5, **characterised in that** lung surfactants obtained by lung lavage are contained.

9. Use according to claim 1 or 2, whereby the medicament is a powder for inhalative application.

10. Use according to claim 1 or 2, whereby the medicament is in fluid form for intratracheal or intrabronchial application.

## Revendications

1. Utilisation d'une combinaison de surfactant pulmonaire et d'au moins un glucocorticostéroïde, à l'exclusion de combinaisons contenant des quantités efficaces de γ-interféron ou de TNF, pour la fabrication d'un médicament destiné au traitement du syndrome de détresse respiratoire du nouveau-né (IRDS) et du syndrome de détresse respiratoire aiguë (ARDS).

2. Utilisation selon la revendication 1, **caractérisée par le fait que** la combinaison est constituée de surfactant pulmonaire et d'au moins un glucocorticostéroïde.

3. Utilisation selon la revendication 1 ou 2, **caractérisée par le fait que** le glucocorticostéroïde est choisi parmi la bétaméthasone, la méthylprédnisolone, la dexaméthasone et/ou le ciclésonide.

4. Utilisation selon la revendication 1 ou 2, **caractérisée par le fait que** le surfactant pulmonaire est un mélange de phospholipides.

5. Utilisation selon la revendication 4, **caractérisée par le fait que** les phospholipides sont des phospholipides naturellement présents dans le surfactant pulmonaire.

6. Utilisation selon la revendication 4, **caractérisé par le fait que** la combinaison contient en outre des protéines de surfactant pulmonaire.

7. Utilisation selon la revendication 6, **caractérisée par** le fait la combinaison contient du SP-B et/ou du SP-C et/ou des dérivés modifiés de ceux-ci.

8. Utilisation selon la revendication 5, **caractérisée par le fait que** le surfactant pulmonaire est obtenu par lavage pulmonaire.

9. Utilisation selon la revendication 1 ou 2, où le médicament se présente sous forme de poudre destinée à l'administration par inhalation.

10. Utilisation selon la revendication 1 ou 2, où le médicament se présente sous forme de liquide destiné à l'administration intratrachéale ou intrabronchique.
